# EUROPÄISCHE PATENTANMELDUNG

(11) **EP 4 144 402 A1**
(43) Veröffentlichungstag der Anmeldung: **08.03.2023**
(21) Anmeldenummer: 22191020.1
(22) Anmeldetag: 18.08.2022
(51) Int. Cl.: A61M 39/28, F16K 7/04, F16K 31/08, F16K 37/00

(54) **QUETSCHVENTILVORRICHTUNG**

(30) Priorität: 03.09.2021 EP 21194899
(71) Anmelder: Levitronix GmbH, 8048 Zürich (CH)
(72) Erfinder: Mayr, Peter, 5400 Baden (CH); Schneeberger, Thomas, 3006 Bern (CH)
(74) Vertreter: IPS Irsch AG

(57) **Zusammenfassung**

Es wird eine Quetschventilvorrichtung vorgeschlagen zum Abklemmen eines Schlauches in einem Fluidsystem, mit einem in einer axialen Richtung (A) bewegbar angeordneten Schliessorgan (2), welches ein Schliessstück (21) zum Abklemmen des Schlauches (100) umfasst, mit einer permanentmagnetischen Halteeinrichtung (5), welche derart ausgestaltet ist, dass sie das Schliessorgan (2) mittels einer permanentmagnetischen Kraft in zwei verschiedenen stabilen Gleichgewichtslagen halten kann, nämlich in einer Offenstellung und in einer Schliessstellung, ohne dass der permanentmagnetischen Halteeinrichtung (5) für das Halten in der jeweiligen Gleichgewichtslage Energie zugeführt werden muss, sowie mit einer elektromagnetischen Betätigungseinrichtung (6) zum Durchführen eines Schaltvorgangs, mit welchem das Schliessorgan (2) aus der Offenstellung in die Schliessstellung oder aus der Schliessstellung in die Offenstellung bewegt wird. Es sind ein Energiespeicher (7) und eine Kontrolleinheit (8) vorgesehen, wobei in dem Energiespeicher (7) eine elektrische Energie speicherbar ist, die mindestens zum Durchführen eines Schaltvorgangs ausreicht, wobei der Kontrolleinheit (8) eine Ruheposition vorgebbar ist, welche die Offenstellung oder die Schliessstellung ist, und wobei die Kontrolleinheit (8) einen Schaltvorgang auslösen kann, mit welchem das Schliessorgan (2) mittels der im Energiespeicher (7) gespeicherten Energie in die Ruheposition gebracht werden kann.

## Beschreibung

Die Erfindung betrifft eine Quetschventilvorrichtung zum Abklemmen eines Schlauches in einem Fluidsystem gemäss dem Oberbegriff des unabhängigen Patentanspruchs.

Fluidsysteme, beispielsweise für biologische Flüssigkeiten, umfassen typischerweise eine Pumpvorrichtung für das zu fördernde Fluid, welche über Schläuche mit einem Kreislauf bzw. mit anderen Komponenten des Fluidsystems, wie beispielsweise Vorratsbehälter oder Filtervorrichtungen verbunden ist. Als Beispiele für solche Fluidsystem seien Anlagen in der Biotechnologie oder in der Pharmaindustrie genannt, die einen Bioreaktor umfassen, in welchem in einer Zellbrühe (cell broth) Proteine oder andere biologische Substanzen hergestellt und aus dem Prozess entnommen werden. Ein anderes Beispiel sind Herz-Lungen-Maschinen, welche z. B. während einer Herzoperation mit dem Blutkreislauf des Patienten verbunden werden, um die Funktion des Herzens zu übernehmen und den Blutkreislauf aufrechtzuerhalten. Dabei ist es sehr wichtig, dass in dem Blut, welches in den Kreislauf des Patienten gefördert wird, möglichst keine Luftblasen vorhanden sind, weil diese ernsthafte Gefährdungen des Patienten darstellen. Daher ist in Herz-Lungen-Maschinen üblicherweise stromabwärts der Pumpe ein Blasendetektor und eine Quetschventilvorrichtung (Pinch Valve System) vorgesehen. Sobald der Blasendetektor eine Luftblase detektiert, muss die Quetschventilvorrichtung möglichst schnell den Schlauch, durch welchen das geförderte Blut in den Körper des Patienten strömt, abklemmen und damit die Blutversorgung zum Patienten unterbrechen, damit die Luftblase nicht in den Körperkreislauf vordringen kann.

Aber auch in anderen Fluidsystemen ist es oft eine Notwendigkeit, dass ein Schlauch möglichst rasch und zuverlässig abgeklemmt werden kann, um eine weitere Durchströmung des Schlauches zu verhindern. Die hierfür verwendeten Quetschventilvorrichtungen dienen somit als Ein/Aus-Schalter für die Strömungsverbindung zwischen Komponenten des Fluidsystems. Quetschventilvorrichtungen zum Abklemmen eines Schlauches sind in zahlreichen Ausführungsformen bekannt. Eine besonders effiziente und zuverlässige Quetschventilvorrichtung wird in der EP-A-1 132 108 offenbart. Diese Quetschventilvorrichtung umfasst ein bewegbar angeordnetes Schliessorgan mit einem Schliessstück zum Abklemmen des Schlauches, eine permanentmagnetische Halteeinrichtung, welche derart angeordnet und ausgestaltet ist, dass sie das Schliessorgan in zwei verschiedenen stabilen Gleichgewichtslagen, nämlich einer Offenstellung und einer Schliessstellung, halten kann, ohne dass der permanentmagnetischen Halteeinrichtung für das Halten in der jeweiligen Gleichgewichtslage Energie zugeführt werden muss, sowie Betätigungsmittel, um das Schliessorgan aus der Offenstellung in die Schliessstellung zu bewegen.

Die permanentmagnetische Halteeinrichtung ist derart ausgestaltet, dass für das Schliessorgan zwei stabile Gleichgewichtslagen existieren, nämlich zum einen die Offenstellung, in welcher ein in die Quetschventilvorrichtung eingelegter Schlauch nicht oder nur wenig geklemmt wird, sodass die Flüssigkeit durch den Schlauch hindurchströmen kann, und zum anderen eine Schliessstellung, in welcher der Schlauch durch das Schliessstück des Schliessorgans abgeklemmt wird, sodass keine Flüssigkeit mehr durch den Schlauch strömen kann. Zum Halten des Schliessorgans in den beiden Gleichgewichtslagen bedarf es keiner elektrischen Energie. Das Schliessorgan wird in den beiden Gleichgewichtslagen rein passiv, nämlich permanentmagnetisch, gehalten, was im Hinblick auf den Energieverbrauch ein ganz erheblicher Vorteil ist. Es handelt sich also um eine bistabile Vorrichtung, welche nur für das Schalten aus einer der Gleichgewichtslagen in die andere Gleichgewichtslage Energie benötigt, nicht aber für das Halten in der jeweiligen stabilen Gleichgewichtslage.

Zudem sind keine Spindelantriebe oder sonstige selbsthemmenden Antriebe zur Betätigung der Quetschventilvorrichtung notwendig, weshalb die Quetschventilvorrichtung konstruktiv einfach und sehr kompakt ist.

Die Betätigungsmittel umfassen eine Spule, welche so angeordnet ist, dass sie auf das Schliessorgan eine in Richtung auf die Schliessstellung oder in Richtung auf die Offenstellung wirkende elektromagnetische Kraft ausüben kann. Durch Aktivieren der Spule wird zusätzlich zur permanentmagnetischen Haltekraft eine elektromagnetische Kraft erzeugt, welche das Schliessorgan so weit aus seiner einen stabilen Gleichgewichtslage auslenkt, dass es seine andere stabile Gleichgewichtslage einnimmt. Die Spule braucht also nur aktiviert zu werden, falls das Schliessorgan aus der Offen- in die Schliessstellung gebracht werden soll oder umgekehrt aus der Schliess- in die Offenstellung.

Auch wenn sich die in der EP-A-1 132 108 offenbarte Quetschventilvorrichtung in der Praxis sehr gut bewährt hat, so besteht Potential für Verbesserungen.

Ausgehend von diesem Stand der Technik ist es daher eine Aufgabe der Erfindung, eine Quetschventilvorrichtung der vorangehend beschriebenen Art weiter zu verbessern. Insbesondere sollen eine höhere Betriebssicherheit und eine hohe Flexibilität realisierbar sein.

Der diese Aufgabe lösenden Gegenstand der Erfindung ist durch die Merkmale des unabhängigen Patentanspruchs gekennzeichnet.

Erfindungsgemäss wird also eine Quetschventilvorrichtung zum Abklemmen eines Schlauches in einem Fluidsystem vorgeschlagen, mit einem in einer axialen Richtung bewegbar angeordneten Schliessorgan, welches ein Schliessstück zum Abklemmen des Schlauches umfasst, mit einer permanentmagnetischen Halteeinrichtung, welche derart ausgestaltet ist, dass sie das Schliessorgan mittels einer permanentmagnetischen Kraft in zwei verschiedenen stabilen Gleichgewichtslagen halten kann, nämlich in einer Offenstellung und in einer Schliessstellung, ohne dass der permanentmagnetischen Halteeinrichtung für das Halten in der jeweiligen Gleichgewichtslage Energie zugeführt werden muss, sowie mit einer elektromagnetischen Betätigungseinrichtung zum Durchführen eines Schaltvorgangs, mit welchem das Schliessorgan aus der Offenstellung in die Schliessstellung oder aus der Schliessstellung in die Offenstellung bewegt wird. Es sind ein Energiespeicher und eine Kontrolleinheit vorgesehen, wobei in dem Energiespeicher eine elektrische Energie speicherbar ist, die mindestens zum Durchführen eines Schaltvorgangs ausreicht, wobei der Kontrolleinheit eine Ruheposition vorgebbar ist, welche die Offenstellung oder die Schliessstellung ist, und wobei die Kontrolleinheit einen Schaltvorgang auslösen kann, mit welchem das Schliessorgan mittels der im Energiespeicher gespeicherten Energie in die Ruheposition gebracht werden kann.

Bei der erfindungsgemässen Quetschventilvorrichtung ist es somit möglich, auch bei einem vollständigen Ausfall der externen Energieversorgung, beispielsweise bei einer Netzunterbrechung, das Schliessorgan der Quetschventilvorrichtung in die vorgebbare Ruheposition zu bringen. Der Energiespeicher der Quetschventilvorrichtung hat in jedem Fall so viel Energie gespeichert, dass auch beim Ausfall der externen Energieversorgung mindestens noch ein Schaltvorgang möglich ist. Es ist also in jedem Fall möglich, auch ohne externe Energieversorgung das Schliessorgan in die gewünschte Ruheposition zu bringen. Dies bedeutet eine enorme Erhöhung der Betriebssicherheit.

Zudem zeichnet sich die erfindungsgemässe Quetschventilvorrichtung durch eine hohe Flexibilität aus, denn die vorgebbare Ruheposition kann die Offenstellung oder die Schliessstellung sein. Abhängig von dem spezifischen Anwendungsfall kann es nämlich wünschenswert oder sogar notwendig sein, dass bei einem Ausfall der externen Energieversorgung das Schliessorgan in die Offenstellung gebracht wird, damit das Fluid den Schlauch durchströmen kann. In anderen Anwendungsfällen kann es wünschenswert oder sogar notwendig sein, dass bei einem Ausfall der externen Energieversorgung das Schliessorgan in die Schliessstellung gebracht wird, damit der Schlauch abgeklemmt wird und ein weiteres Strömen des Fluids durch den Schlauch zuverlässig verhindert wird. Die Auswahl, ob die Ruheposition die Offenstellung oder die Schliessstellung ist, kann beispielsweise elektronisch, elektromechanisch oder mittels Software erfolgen.

Gemäss einer besonders bevorzugten Ausführungsform sind das Schliessorgan, die Halteeinrichtung, die Betätigungseinrichtung, der Energiespeicher und die Kontrolleinrichtung in einem gemeinsamen Gehäuse angeordnet, wobei das Gehäuse einen Anschluss zum Verbinden mit einer externen Energiequelle aufweist. Diese Ausführungsform ist äusserst kompakt und besonders einfach in der Handhabung. Der Anschluss zum Verbinden mit einer externen Energiequelle kann zusätzlich für Steuer- oder Signalleitungen verwendet werden, mit denen die Quetschventilvorrichtung angesteuert oder programmiert werden kann.

Vorzugsweise ist in dem gemeinsamen Gehäuse ein Überwachungssensor vorgesehen, mit welchem eine Unterbrechung der Energieversorgung durch die externe Energiequelle detektierbar ist. Der im Gehäuse integrierte Überwachungssensor kann also detektieren, wenn die Energieversorgung durch die externe Energiequelle komplett ausfällt oder nicht mehr ausreichend ist. Beispielsweise kann der Überwachungssensor eine Unterbrechung der Versorgungsspannung oder des Netzstroms detektieren, welche von der externen Energiequelle bereitgestellt wird. Sobald eine Unterbrechung der Versorgungsspannung bzw. des Stroms detektiert wird, wird diese Information an die Kontrolleinheit weitergegeben. Die Kontrolleinheit überprüft, ob das Schliessorgan in der Offenstellung oder in der Schliessstellung ist, und welches die vorgegebenen Ruheposition für das Schliessorgan ist. Falls sich das Schliessorgan nicht in der gewünschten Ruheposition befindet, so löst die Kontrolleinheit einen Schaltvorgang aus, welcher das Schliessorgan in die Ruheposition bringt. Im Energiespeicher ist in jedem Fall ausreichend Energie gespeichert, um mindestens einen Schaltvorgang durchzuführen, auch wenn die externe Energiequelle keine Energie mehr zur Verfügung stellen kann.

In einer bevorzugten Ausführungsform umfasst die permanentmagnetische Halteeinrichtung einen permanentmagnetischen Ring, welcher das Schliessorgan umgibt. Mit diesem permanentmagnetischen Ring kann das Schliessorgan mittels permanentmagnetischer Kräfte in jeder der beiden stabilen Gleichgewichtslagen gehalten werden, ohne dass der Quetschventilvorrichtung Energie z.B. in Form von elektrischem Strom zugeführt werden muss. Vorzugsweise ist die Halteeinrichtung auch ohne Federn zum Halten des Schliessorgans in den stabilen Gleichgewichtslagen ausgestaltet.

Gemäss einer bevorzugten Ausgestaltung ist der Energiespeicher als Zwischenkreis für die Energieversorgung der Betätigungseinrichtung ausgestaltet. Der Energiespeicher kann dabei mindestens einen Kondensator umfassen, dessen Kapazität ausreichend gross ist, um die Betätigungseinrichtung für mindestens einen Schaltvorgang mit elektrischer Energie zu versorgen, falls die externe Energieversorgung ausgefallen ist. Durch diesen gross ausgestalteten Energiespeicher kann vorteilhafterweise auch die Energiequelle, welche den Zwischenkreis mit elektrischer Energie versorgt, kleiner dimensioniert werden.

Ferner ist es bevorzugt, dass ein Positionssensor zur Ermittlung der Position des Schliessorgans vorgesehen ist.

Eine weitere vorteilhafte Massnahme besteht darin, dass ein Messsensor zur Ermittlung der im Energiespeicher gespeicherten elektrischen Energie vorgesehen ist.

Auch ist es bevorzugt, dass ein Stromsensor zur Ermittlung eines Schaltstroms vorgesehen ist, wobei der Schaltstrom derjenige Strom ist, der für den Schaltvorgang benötigt wird.

Der Schaltstrom kann beispielsweise ein Schliessstrom sein, wobei der Schliessstrom derjenige Strom ist, der für den Schaltvorgang aus der Offenstellung in die Schliessstellung benötigt wird. Der Schaltstrom kann beispielsweise ein Öffnungsstrom sein, wobei der Öffnungsstrom derjenige Strom ist, der für den Schaltvorgang aus der Schliessstellung in die Offenstellung benötigt wird.

Mit dem Stromsensor kann der in die Betätigungseinrichtung eingespeiste Strom über den kompletten Schaltvorgang, also über die gesamte Hubbewegung des Schliessorgans aus der Offen- in die Schliessstellung - oder umgekehrt - bestimmt werden. Diese Information kann beispielsweise dazu genutzt werden festzustellen, ob ein Schlauch in die Quetschventilvorrichtung eingelegt ist oder nicht.

Insbesondere ist es bei dieser Ausgestaltung möglich, dass eine Schlaucherkennung anhand des Schaltstroms durchführbar ist. Hierzu können beispielsweise in der Kontrolleinheit in einer Zuordnungstabelle (Lookup Tabelle) Zusammenhänge gespeichert sein, welcher Strom für welchen Schlauchtyp benötigt wird, um einen Schaltvorgang aus der Offenstellung in die Schliessstellung und/oder aus der Schliessstellung in die Offenstellung durchzuführen. Der Schlauchtyp kann dabei z.B. Informationen über das jeweilige Material des Schlauches, seinen Durchmesser, seine Wandstärke und gegebenenfalls noch weitere Parameter enthalten. Anhand des messtechnisch erfassten Schaltstroms und gegebenenfalls dem Signal des Positionssensors ist es dann möglich, den in die Quetschventilvorrichtung eigelegten Schlauch zu identifizieren.

Gemäss einer bevorzugten Ausgestaltung sind im Gehäuse zwei laterale Öffnungen zum Aufnehmen eines Schlauches vorgesehen, wobei ein Verschlussdeckel vorgesehen ist, der gelenkig mit dem Gehäuse verbunden ist, und zwischen einer ersten Position und einer zweiten Position hin und her bewegbar ist, wobei in der ersten Position der Schlauch in die lateralen Öffnungen einlegbar ist, und wobei in der zweiten Position der Schlauch relativ zum Gehäuse der Quetschventilvorrichtung fixiert ist.

Dabei ist vorzugsweise im Verschlussdeckel ein Schlauchquetschelement zum Zusammenwirken mit dem Schliessstück des Schliessorgans angeordnet, derart, dass der Schlauch zwischen dem Schliessstück und dem Schlauchquetschelement einklemmbar ist, wenn der Verschlussdeckel in der zweiten Position ist.

Ferner ist es eine bevorzugte Massnahme, dass das Schlauchquetschelement austauschbar ausgestaltet und angeordnet ist, sodass die Quetschventilvorrichtung in einfacher Weise an verschiedene Schlauchtypen und insbesondere an verschiedene Aussendurchmesser und/oder Innendurchmesser des Schlauches anpassbar ist.

Dabei ist es eine bevorzugte Ausgestaltung, dass im Verschlussdeckel eine Ausnehmung vorgesehen ist, in welche das Schlauchquetschelement einschiebbar ist. Dadurch kann das Schlauchquetschelement in besonders einfacher und schneller Weise und insbesondere ohne die Verwendung eines Werkzeugs ausgetauscht werden.

Ferner ist es bevorzugt, dass ein in der axialen Richtung bewegbares Schutzelement vorgesehen ist, welches durch eine Feder belastet ist, derart, dass das Schutzelement die beiden lateralen Öffnungen überdeckt, wenn der Verschlussdeckel in der ersten Position oder in der zweiten Position ist, und kein Schlauch in die Quetschventilvorrichtung eingelegt ist. Dieses Schutzelement dient insbesondere als Fingerschutz für das Bedienpersonal, damit es nicht zu einem unbeabsichtigten Eingreifen in die lateralen Öffnungen kommt, beispielsweise, falls kein Schlauch in die Quetschventilvorrichtung eingelegt ist, oder beim Einlegen eines Schlauches. Beim Einlegen eines Schlauches wird das Schutzelement mittels des Schlauchs gegen die Kraft der Feder in axialer Richtung bewegt, bis der Schlauch in den beiden lateralen Öffnungen liegt. Anschliessend wird der Verschlussdeckel in die zweite Position gebracht, sodass der Schlauch zwischen dem Schlauchquetschelement und dem Schliessstück fixiert ist.

Als weitere vorteilhafte Massnahme kann am Gehäuse ein Sicherungselement vorgesehen werden, mit welchem der Verschlussdeckel in der zweiten Position fixiert werden kann. Hierdurch lässt sich ein unbeabsichtigtes Entfernen des Schlauches aus der Quetschventilvorrichtung verhindern.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der teilweise schematischen Zeichnung zeigen:
- Fig. 1:: ein Ausführungsbeispiel einer erfindungsgemässen Quetschventilvorrichtung in einer perspektivischen Ansicht,
- Fig. 2:: eine Schnittdarstellung des Ausführungsbeispiels in einem Schnitt in axialer Richtung,
- Fig. 3:: eine perspektivische Darstellung des Verschlussdeckels des Ausführungsbeispiels und der lateralen Öffnungen zur Aufnahme eines Schlauches,
- Fig. 4:: eine perspektivische Darstellung des Schutzelements des Ausführungsbeispiels,
- Fig. 5:: eine schematische Darstellung des Ausführungsbeispiels,
- Fig. 6:: mehrere Diagramme zur Erläuterung des Betriebs des Ausführungsbeispiels,
- Fig. 7:: wie Fig. 5, jedoch für eine erste Variante des Ausführungsbeispiels, und
- Fig. 8:: wie Fig. 5, jedoch für eine zweite Variante des Ausführungsbeispiels.

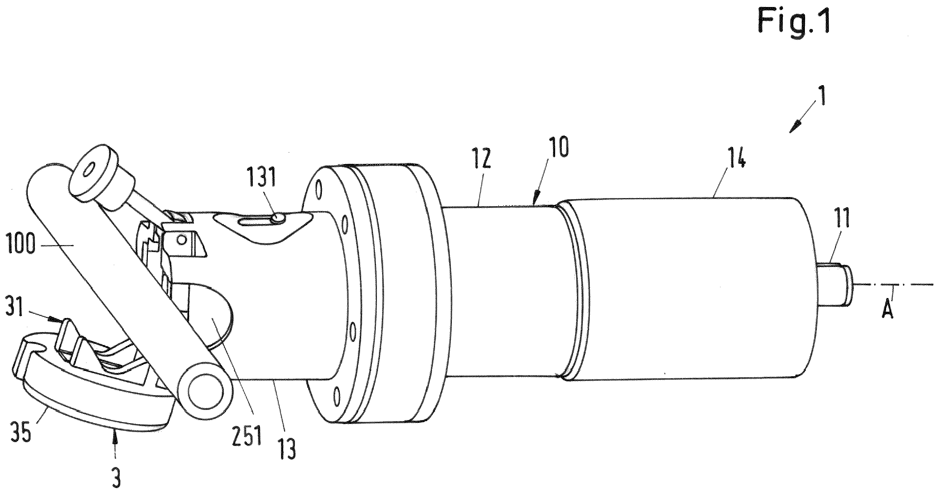

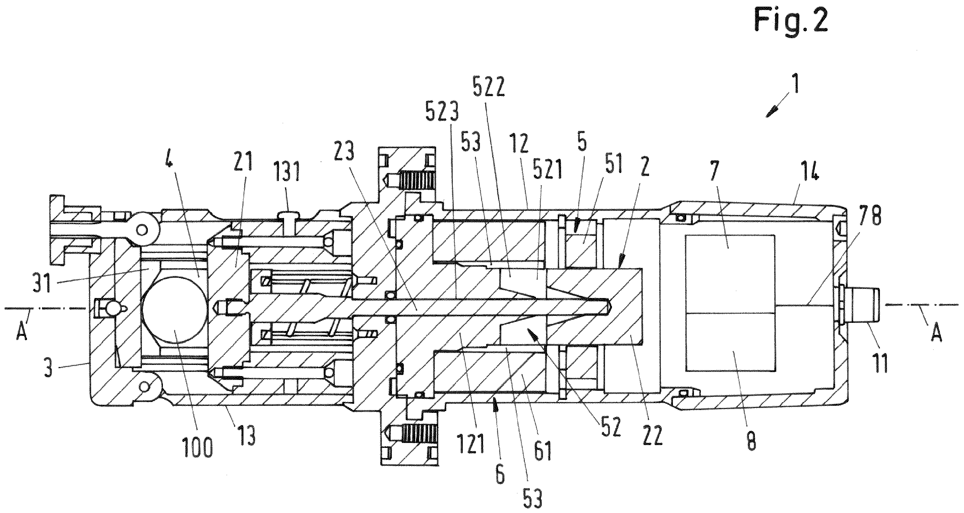

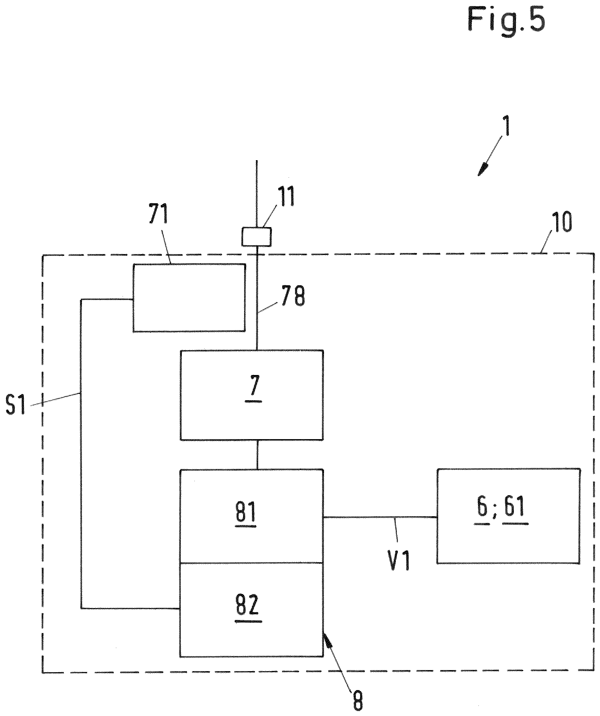

Fig. 1 zeigt eine perspektivische Darstellung eines Ausführungsbeispiels einer erfindungsgemässen Quetschventilvorrichtung, die gesamthaft mit dem Bezugszeichen 1 bezeichnet ist. Zum besseren Verständnis zeigen Fig. 2 eine Schnittdarstellung des Ausführungsbeispiels in einem Schnitt in axialer Richtung A und Fig. 5 eine schematische Darstellung des Ausführungsbeispiels.

Die Quetschventilvorrichtung 1 dient zum Abklemmen eines Schlauches 100 in einem Fluidsystem, wobei der Schlauch 100 in die Quetschventilvorrichtung 1 einlegbar und in dieser fixierbar ist.

Die Quetschventilvorrichtung 1 umfasst ein Schliessorgan 2, welches in einer axialen Richtung A bewegbar angeordnet ist. Das Schliessorgan 2 umfasst ein Schliessstück 21 zum Abklemmen des Schlauches 100, welches mit einem Schlauchquetschelement 31 zusammenwirken kann, dass in einem Verschlussdeckel 3 der Quetschventilvorrichtung 1 angeordnet ist. Das Schliessstück 21 und das Schlauchquetschelement 31 begrenzen bezüglich der axialen Richtung eine Aufnahme 4 für den Schlauch 100, die zwischen dem Schliessstück 21 und dem Schlauchquetschelement 31 angeordnet ist, und in welche der Schlauch 100 einlegbar ist.

Die Quetschventilvorrichtung 1 umfasst ferner eine permanentmagnetische Halteeinrichtung 5, eine elektromagnetische Betätigungseinrichtung 6, einen Energiespeicher 7 und eine Kontrolleinheit 8.

Das Schliessorgan 2, die Halteeinrichtung 5, die Betätigungseinrichtung 6, der Energiespeicher 7 und die Kontrolleinheit 8 sind in einem gemeinsamen Gehäuse 10 der Quetschventilvorrichtung 1 angeordnet, wobei an dem Gehäuse ein Anschluss 11 vorgesehen ist, mit welchem die Quetschventilvorrichtung 1 mit einer externen Energiequelle verbindbar ist. Der Anschluss 11 kann zusätzlich für Steuer- oder Signalleitungen verwendet werden, mit denen die Quetschventilvorrichtung 1 angesteuert oder programmiert werden kann, oder Daten und andere Informationen aus der Quetschventilvorrichtung 1 exportiert werden können.

Die permanentmagnetische Halteeinrichtung 5 ist derart ausgestaltet, dass sie das Schliessorgan 2 mittels einer permanentmagnetischen Kraft in zwei verschiedenen stabilen Gleichgewichtslagen halten kann, nämlich in einer Offenstellung und in einer Schliessstellung, ohne dass der permanentmagnetischen Halteeinrichtung 5 für das Halten in der jeweiligen Gleichgewichtslage Energie zugeführt werden muss. Dazu umfasst die Halteeinrichtung 5 bei diesem Ausführungsbeispiel einen permanentmagnetischen Ring 51, welcher um das Schliessorgan 2 herum angeordnet ist.

In der in Fig. 2 dargestellten Offenstellung ist der in die Aufnahme 4 eingelegte Schlauch 100 nicht oder nur sehr wenig geklemmt, sodass die Flüssigkeit bzw. das Fluid durch den Schlauch 100 hindurchströmen kann, der Schlauch 100 aber fixiert ist. In der Schliessstellung wird der Schlauch 100 derart zwischen dem Schliessstück 21 und dem Schlauchquetschelement 31 eingeklemmt, dass keine Flüssigkeit mehr durch den Schlauch 100 hindurchströmen kann. In der Offenstellung (Fig. 2) ist der Strömungsquerschnitt für die Flüssigkeit im Schlauch 100 maximal. Zum Schliessen wird das Schliessorgan 2 und damit auch das Schliessstück 21 darstellungsgemäss (Fig. 2) nach links bewegt und presst dadurch den Schlauch 100 derart zusammen, dass der Strömungsquerschnitt null wird. In dieser Schliessstellung des Schliessorgans 2 ist der Schlauch 100 dann abgeklemmt.

Die elektromagnetischen Betätigungseinrichtung 6 ist zum Durchführen eines Schaltvorgangs ausgestaltet, mit welchem das Schliessorgan 2 aus der Offenstellung in die Schliessstellung oder aus der Schliessstellung in die Offenstellung bewegt werden kann. Dazu umfasst die elektromagnetische Betätigungseinrichtung 6 mindestens eine Spule 61, die von der Kontrolleinheit 8 zur Durchführung eines Schaltvorgangs ansteuerbar ist. Für einen Schaltvorgang wird mittels der Spule 61 auf das Schliessorgan 2 zusätzlich eine elektromagnetische Kraft ausgeübt, welche stark genug ist, um das Schliessorgan so weit aus seiner einen stabilen Gleichgewichtslage (z. B. die Offenstellung) auszulenken, dass das Schliessorgan 2 die andere seiner beiden stabilen Gleichgewichtslagen (z. B. die Schliessstellung) einnimmt.

Ein wesentlicher Aspekt ist es, dass die permanentmagnetische Halteeinrichtung 5 das Schliessorgan 2 in beiden Gleichgewichtslagen (Offenstellung und Schliessstellung) halten kann, ohne dass hierfür Energie, z.B. elektrischer Strom zugeführt werden muss. Während des Haltens benötigt die Quetschventilvorrichtung 1 keine elektrische Energie, weshalb sie enorm sparsam bezüglich des Energieverbrauchs in den beiden Gleichgewichtslagen ist.

Die permanentmagnetische Halteeinrichtung 5 (siehe Fig. 2) ist in einem Aktuatorgehäuse 12 angeordnet, das einen Teil des gemeinsamen Gehäuses 10 bildet. Das Aktuatorgehäuse 12 ist aus einem ferromagnetischen Material gefertigt. Die Halteeinrichtung 5 weist einen zentralen Durchlass 52 zur Aufnahme des Schliessorgans 2 auf. Der Durchlass 52 erstreckt sich in axialer Richtung A und weist ein ersten zylindrischen Bereich 521 auf, an den sich ein konischer Bereich 522 anschliesst, der dann in einen zweiten zylindrischen Bereich 523 übergeht, dessen Durchmesser kleiner ist als der Durchmesser des ersten zylindrischen Bereichs 521. Der konische Bereich 522 und der zweite zylindrische Bereich 523 werden durch einen Fortsatz 121 des Aktuatorgehäuses 12 begrenzt, welches als magnetischer Rückschluss zur Führung des magnetischen Flusses dient und ferromagnetisch ausgestaltet ist. Dabei weist der Fortsatz 121 eine konische Begrenzungsfläche auf, die schräg zur axialen Richtung A verläuft und mit einer ebenfalls konisch ausgestalteten Fläche des Schliessorgans 2 zusammenwirken kann. In den ersten zylindrischen Bereich 521 ist eine Hülse 53 eingesetzt, die aus einem nichtferromagnetischen Material, beispielsweise Kunststoff hergestellt ist.

Das Schliessorgan 2 umfasst einen zylindrisch ausgestalteten Kopf 22, dessen Aussendurchmesser an den Innendurchmesser der Hülse 53 im ersten zylindrischen Bereich 521 des Durchlasses 52 angepasst ist, sodass der Kopf 22 in die Hülse 53 eintauchen kann und durch diese geführt ist. Der Kopf 22 ist aus einem ferromagnetischen Material gefertigt und weist an seinem darstellungsgemäss (Fig. 2) linken Ende die konisch ausgestaltete Fläche aus, welche mit der konischen Begrenzungsfläche des Fortsatzes 121 zusammenwirkt. An diesem Ende des Kopfes 22 ist ferner ein Stab 23 befestigt, welcher sich in axialer Richtung A durch den zweiten zylindrischen Bereich 523 des Durchlasses 52 hindurch erstreckt und an dessen darstellungsgemäss (Fig. 2) linken Ende das Schliessstück 21 fixiert ist.

In dem Aktuatorgehäuse 12 ist ferner der permanentmagnetischen Ring 51 vorgesehen, der so angeordnet ist, dass er den Kopf 22 des Schliessorgans 2 umschliesst.

In axialer Richtung A benachbart zu dem permanentmagnetischen Ring 51 ist in dem Aktuatorgehäuse 12 die Spule 61 vorgesehen, welche als Betätigungsmittel dient, um das Schliessorgan 2 aus seiner Offenstellung in seine Schliessstellung zu bewegen bzw. umgekehrt. Die Spule 61 umschliesst den Durchlass 52, sodass die Spulenachse in axialer Richtung A verläuft. Die Spule 61 kann also, wenn sie mit Strom gespeist wird, eine in axialer Richtung A gerichtete elektromagnetische Kraft auf das Schliessorgan 2 ausüben, wobei diese elektromagnetische Kraft je nach Stromrichtung darstellungsgemäss (Fig. 2) nach rechts oder nach links gerichtet sein kann.

Die Spule 61 grenzt mit ihrem dem permanentmagnetischen Ring 51 abgewandten axialen Ende an den Fortsatz 121 des Aktuatorgehäuses 12 an. Die Spule 61 ist ferner mit der Kontrolleinheit 8 signalverbunden, sodass die Kontrolleinheit 8 die Spule 61 mit elektrischer Energie versorgen und einen Schaltvorgang auslösen kann.

Bezüglich der Existenz der beiden stabilen Gleichgewichtslagen, in denen das Schliessorgan 2 durch permanentmagnetische Kräfte ohne weitere Energiezufuhr gehalten werden kann, sowie der Schaltvorgänge, bei denen das Schliessorgan 2 mittels von der Spule 61 erzeugter elektromagnetischer Kräfte aus der Schliessstellung in die Offenstellung bzw. aus der Offenstellung in die Schliessstellung bewegt werden kann, wird auf die bereits erwähnte EP-A-1 132 108 und die dort zu findenden ausführlichen Erläuterungen verwiesen.

Erfindungsgemäss ist der Energiespeicher 7 so ausgestaltet, dass in dem Energiespeicher eine elektrische Energie speicherbar ist, die mindestens zum Durchführen eines Schaltvorgangs ausreicht. Ferner ist der Kontrolleinheit 8 eine Ruheposition vorgebbar, welche die Offenstellung oder die Schliessstellung ist, wobei die Kontrolleinheit 8 einen Schaltvorgang auslösen kann, mit welchem das Schliessorgan 2 mittels der im Energiespeicher 7 gespeicherten Energie in die Ruheposition gebracht werden kann.

Dies wird im Folgenden anhand des Ausführungsbeispiels und unter Bezugnahme auf die Fig. 1, Fig.2 und Fig. 5 näher erläutert.

Bei dem hier beschriebenen Ausführungsbeispiel ist die Quetschventilvorrichtung 1 als ein voll integriertes, bistabiles System ausgestaltet, bei dem für die Schaltvorgänge bzw. für das Halten in jeder der beiden stabilen Gleichgewichtslagen keine Rückstellfedern vorgesehen sind. Das gemeinsame Gehäuse 10 umfasst das Aktuatorgehäuse 12, ein Verschlussgehäuse 13 und ein Schutzgehäuse 14, wobei das Aktuatorgehäuse 12 bezüglich der axialen Richtung A zwischen dem Verschlussgehäuse 13 und dem Schutzgehäuse 14 angeordnet ist. Das Aktuatorgehäuse 12, das Verschlussgehäuse 13 und das Schutzgehäuse 14 sind fest miteinander verbunden, beispielsweise durch Schrauben oder andere Befestigungsmittel, sodass sie das gemeinsame Gehäuse 10 bilden.

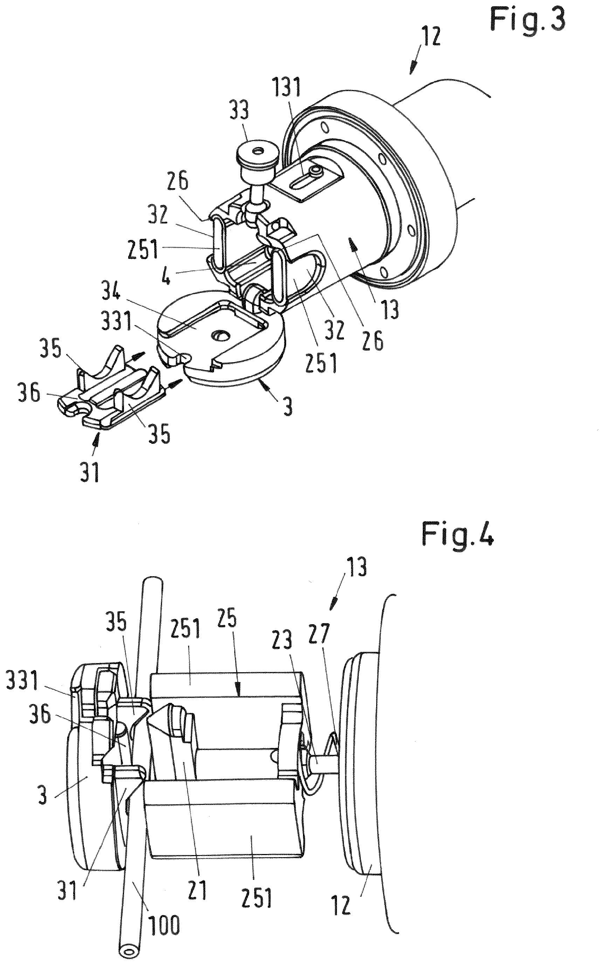

An dem axialen Ende des Verschlussgehäuses 13, welcher dem Aktuatorgehäuse 12 abgewandt ist, weist das Verschlussgehäuse 13 den Verschlussdeckel 3 auf. Der Verschlussdeckel 3 ist gelenkig mit dem Verschlussgehäuse 13 verbunden und zwischen einer ersten Position und einer zweiten Position hin und her bewegbar. In der ersten Position (siehe auch Fig. 3) ist der Schlauch 100 in die Aufnahme 4 einlegbar. In der zweiten Position (siehe auch Fig. 4) ist der Schlauch 100 relativ zum gemeinsamen Gehäuse 10 fixiert, nämlich zwischen dem Schlauchquetschelement 31 und dem Schliessstück 21. Vorzugsweise ist am Verschlussgehäuse 13 eine Anzeige 131 vorgesehen, die von aussen erkennbar anzeigt, ob sich das Schliessorgan 2 in seiner Offenstellung oder in seiner Schliessstellung befindet.

Im Schutzgehäuse 14 sind der Energiespeicher 7 und die Kontrolleinheit 8 angeordnet. Am Schutzgehäuse 14 ist ferner der Anschluss 11 vorgesehen, über welchen die Quetschventilvorrichtung 1 mit einer externen Energiequelle (nicht dargestellt) verbindbar ist. Über den Anschluss 11 kann ferner die Ansteuerung und/oder die Programmierung der Kontrolleinheit 8 erfolgen. Der Anschluss 11 ist über eine elektrische Verbindung 78 mit dem Energiespeicher 7 und/oder der Kontrolleinheit 8 verbunden.

Wie dies in Fig. 5 dargestellt ist, umfasst die Kontrolleinheit 8 eine Leistungseinheit 81 und eine Steuereinheit 82. Die Leistungseinheit 81 umfasst insbesondere die Leistungselektronik, welche die Spule 61 der Betätigungseinrichtung 6 mit Energie, insbesondere mit Strom, versorgt, falls ein Schaltvorgang durchgeführt werden soll. Hierzu ist die Leistungseinheit 81 über eine Verbindung V1 mit der Spule 61 verbunden. Die Steuereinheit 82 umfasst die Steuerungen oder die Regelungen für den Betrieb der Quetschventilvorrichtung 1 sowie optional Speicher-, Verarbeitungs- oder Auswertemodule für den Betrieb der Quetschventilvorrichtung 1.

Besonders bevorzugt ist der Energiespeicher 7 als Zwischenkreis für die Energieversorgung der Betätigungseinrichtung 6 ausgestaltet. Der Energiespeicher 7 ist einerseits über die elektrische Verbindung 78 und den Anschluss 11 mit der externen Energiequelle verbindbar und andererseits über die Verbindung V1 mit der Spule 61 der Betätigungseinrichtung 6 verbunden. Die externe Energiequelle ist beispielsweise eine Versorgungsspannung z. B. die Netzspannung, oder eine andere Spannungsquelle oder eine Stromquelle.

Der Energiespeicher 7 umfasst besonders bevorzugt mindestens einen Kondensator zum Speichern von elektrischer Energie, wobei der Kondensator oder - im Falle mehrerer Kondensatoren - die Gesamtheit der Kondensatoren - eine Kapazität aufweist, die gross genug ist, dass auch bei einem Ausfall der Energieversorgung durch die externe Energiequelle im Energiespeicher 7 genügend elektrische Energie gespeichert ist, um mindestens einen Schaltvorgang auszuführen. Die im Energiespeicher 7 gespeicherte Energie ist vorzugsweise grösser als das Wegintegral über die Kraft entlang der Hubbewegung des Schliessorgans 2 von der Offenstellung in die Schliessstellung.

Die Leistungselektronik in der Leistungseinheit 81 kann in jeder an sich bekannten Art ausgestaltet sein, die zur Ansteuerung der Spule 61 geeignet ist. Die Leistungselektronik kann beispielsweise als H-Brücke mit Überspannungsschutz ausgestaltet sein, wobei die H-Brücke mit der von dem Energiespeicher 7 bereitgestellten Zwischenkreisspannung versorgt wird.

Vorzugsweise ist in dem gemeinsamen Gehäuse 10 ein Überwachungssensor 71 vorgesehen, mit welchem eine Unterbrechung der Energieversorgung detektierbar ist, das heisst ein Wegfall der von der externen Energiequelle gelieferten Energie. Grundsätzlich könnte auch ein externer Sensor vorgesehen sein, der ein Signal an die Kontrolleinheit 8 übermittelt, falls es zu einer Störung an der externen Energiequelle kommt, jedoch ist der intern im gemeinsamen Gehäuse 10 vorgesehene Überwachungssensor 71 bevorzugt. Der Überwachungssensor 71 ist bevorzugt im Schutzgehäuse 14 angeordnet und über eine Signalleitung S1 mit der Kontrolleinheit 8, genauer gesagt mit der Steuereinheit 82 der Kontrolleinheit 8 signalverbunden. Der Überwachungssensor 71 ist vorzugsweise als Stromsensor oder als Spannungssensor ausgestaltet und so angeordnet, dass er einen Fehler in der Versorgung durch die externe Energiequelle detektieren kann. Ein Fehler in der Versorgung kann beispielsweise ein Netzunterbruch sein oder ein Zusammenbruch der Versorgungsspannung, z.B. der Netzspannung oder ein fehlender Stromfluss.

Für den Betrieb der Quetschventilvorrichtung 1 wird der Schlauch 100 in die Aufnahme 4 eingelegt, und der Verschlussdeckel 3 wird in die zweite Position gebracht, in welcher der Schlauch zwischen dem Schliessstück 21 und dem Schlauchquetschelement 31 fixiert ist. Der Steuereinheit 82 der Kontrolleinheit 8 wird die gewünschte Ruheposition vorgegeben, welche die Offenstellung oder die Schliessstellung ist. Somit wird der Kontrolleinheit 8 vorgegeben, ob bei einem Wegfall der externen Energieversorgung das Schliessorgan 2 in die Offenstellung oder in die Schliessstellung gebracht werden soll.

Wenn nun mittels des Überwachungssensors 71 ein Wegfall der externen Energieversorgung, also beispielsweise ein Fehlen der Versorgungsspannung, detektiert wird, so wird diese Information an die Steuereinheit 82 der Kontrolleinheit 8 übermittelt. Die Kontrolleinheit 8 überprüft, ob sich das Schliessorgan 2 in der vorgegebenen Ruheposition befindet. Falls ja, ist es nicht notwendig, weitere Schritte zu unternehmen, weil es keiner Energiezufuhr bedarf, um das Schliessorgan 2 in der Ruheposition zu halten. Falls sich das Schliessorgan 2 nicht in der vorgegebenen Ruheposition befindet, sendet die Steuereinheit 82 einen Befehl an die Leistungseinheit 81, dass ein Schaltvorgang durchgeführt werden muss. Die für diesen Schaltvorgang benötigte elektrische Energie ist im Energiespeicher 7 vorhanden, sodass die Betätigungseinrichtung 6 das Schliessorgan 2 in die Ruheposition bewegen kann. Sobald das Schliessorgan 2 in der Ruheposition ist, bedarf es keiner weiteren Energiezufuhr mehr, um das Schliessorgan 2 in der Ruheposition zu halten.

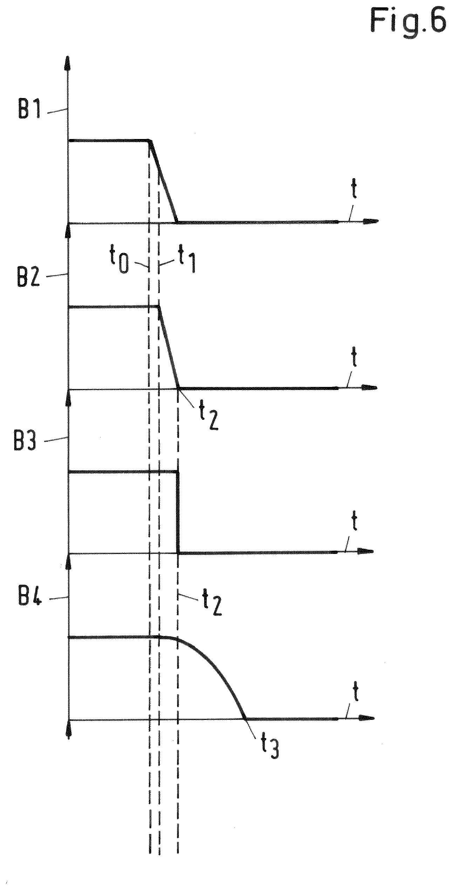

Dieser Betrieb wird in Fig. 6 beispielshaft durch mehrere schematische Diagramme veranschaulicht, bei denen auf der horizontalen Achse jeweils die Zeit t aufgetragen ist. Auf der vertikalen Achse sind von oben nach unten gesehen folgende Grössen aufgetragen: die die von der externen Energiequelle bereitgestellte Versorgungsspannung B1, die Erkennung der Netzunterbrechung B2 (Wegfall der externen Energieversorgung bzw. der Versorgungsspannung), die Aktion der Steuereinheit B3, die Energie B4 im Energiespeicher 7.

Das in Fig. 6 veranschaulichten Beispiel bezieht sich auf den Fall, dass sich das Schliessorgan 2 beim Wegfall der externen Energieversorgung - hier beispielsweise eine Netzunterbrechung, bei welcher die Versorgungsspannung wegfällt - nicht in der Ruheposition, sondern in der anderen der beiden stabilen Gleichgewichtslagen befindet.

Zum Zeitpunkt t₀ kommt es zu einem Unterbruch bzw. zu einem Wegfall der Versorgungsspannung B1. Zum Zeitpunkt t₁ erkennt der Überwachungssensor 71 den Ausfall der externen Energieversorgung B2 und übermittelt diese Information an die Steuereinheit 82. Die Aktion der Steuereinheit B3 erfolgt zum Zeitpunkt t₂, nämlich das Veranlassen eines Schaltvorgangs mittels der im Kondensator des Energiespeichers 7 gespeicherten elektrischen Energie.

Durch den Schaltvorgang nimmt die im Energiespeicher 7 gespeicherte Energie B4 ab. Beim Zeitpunkt t₃ ist der Schaltvorgang beendet, und das Schliessorgan 2 hat die vorgegebene Ruheposition erreicht.

Es versteht sich, dass die im untersten Diagramm der Fig. 6 beispielhaft dargestellte Energie B4, die im Energiespeicher 7 gespeichert ist, zum Zeitpunkt t₃ nicht den Wert null erreichen muss, sondern auch noch einen von null verschiedenen Wert haben kann. Das heisst, es ist durchaus möglich dass der Energiespeicher 7 zum Zeitpunkt t₃ nicht vollständig entladen ist, sondern noch Energie im Energiespeicher 7 gespeichert sein kann.

Im Folgenden wird anhand der Fig. 3 und der Fig. 4 eine bevorzugte Ausgestaltung des Verschlussgehäuses 13 mit dem Verschlussdeckel 3 erläutert. Fig. 3 zeigt eine perspektivische Darstellung des Verschlussgehäuses 13 mit dem Verschlussdeckel 3 in der ersten Position, in welcher der Schlauch 100 in die Aufnahme 4 der Quetschventilvorrichtung 1 einlegbar bzw. aus dieser entfernbar ist (in Fig. 3 ist der Schlauch nicht dargestellt). Fig. 4 zeigt den Verschlussdeckel 3 in der zweiten Position, in welcher der Schlauch 100 in der Aufnahme 4 fixiert ist. Dabei ist der Schlauch 100 zwischen dem Schlauchquetschelement 31 und dem Schliessstück 21 eingeklemmt. Zum besseren Verständnis ist in Fig. 4 von dem Verschlussgehäuse 13 nur der Verschlussdeckel 3 dargestellt; der Rest ist entfernt, um einen Blick in das Innere zu ermöglichen.

Im Verschlussgehäuse 13 sind zwei laterale Öffnungen 32 seitlich der Aufnahme 4 vorgesehen, durch welche der Schlauch 100 das Verschlussgehäuse 13 verlassen kann, wenn der Verschlussdeckel 3 in der zweiten Position ist. In dieser zweiten Position des Verschlussdeckels 3 bilden die lateralen Öffnungen 32 also die beiden Durchlässe, an welchen der Schlauch 100 das gemeinsame Gehäuse 10, genauer gesagt das Verschlussgehäuse 13 des gemeinsamen Gehäuses 10, durchdringt.

Der Verschlussdeckel 3 ist am axialen Ende des Verschlussgehäuses 13 angeordnet und gelenkig mit dem Verschlussgehäuse 13 verbunden, sodass der Verschlussdeckel 3 aus der ersten Position (Fig. 3) um etwa 90° in die zweite Position (Fig. 4) geschwenkt bzw. gekippt werden kann. Ferner ist am Verschlussgehäuse 13 ein Sicherungselement 33 (Fig. 3) vorgesehen, mit welchem der Verschlussdeckel 3 in der zweiten Position fixiert werden kann, sodass ein unbeabsichtigtes Öffnen des Verschlussdeckels 3 - also ein unbeabsichtigtes Verlassen der zweiten Position - sicher vermieden werden kann. Das Sicherungselement 33 ist beispielsweise als Rändelschraube ausgestaltet, welche mit einer Nut 331 im Verschlussdeckel 3 zusammenwirken kann, falls der Verschlussdeckel 3 in der zweiten Position ist. Die Rändelschraube wird in der zweiten Position des Verschlussdeckels von der Nut 331 aufgenommen. Durch Festziehen der Rändelschraube lässt sich der Verschlussdeckel 3 in der zweiten Position fixieren bzw. sichern. Es versteht sich, dass natürlich auch andere Ausgestaltungen des Sicherungselements 33 möglich sind.

Gemäss einer besonders bevorzugten Ausführungsform ist das Schlauchquetschelement 31 austauschbar im Verschlussdeckel 3 angeordnet, sodass das Schlauchquetschelement 31 in einfacher Weise gegen ein anderes Schlauchquetschelement 31, beispielsweise eines anderer Grösse, austauschbar ist.

Dazu umfasst der Verschlussdeckel 3 vorzugsweise eine Ausnehmung 34, in welche das Schlauchquetschelement 31 einschiebbar ist. In Fig. 3 ist das Schlauchquetschelement 31 zum besseren Verständnis ausserhalb von der Ausnehmung 34 dargestellt. Das Schlauchquetschelement 31 kann, wie dies die beiden Pfeile ohne Bezugszeichen in Fig. 3 andeuten, in einfacher Weise in die Ausnehmung 34 im Verschlussdeckel 3 eingeschoben bzw. aus dieser entfernt werden. Vorzugsweise umfasst die Ausnehmung 34 zwei seitliche Nuten, welche das Schlauchquetschelement 31 umfassen und führen, sodass das Schlauchquetschelement 31 in der Ausnehmung 34 gesichert ist.

Das Schlauchquetschelement 31 umfasst zwei seitliche Schlauchauflagen 35, welche jeweils flach, gerundet U-förmig oder V-förmig ausgestaltet sind, sodass der Schlauch 100 teilweise von den Schlauchauflagen 35 umfasst wird. Das Schlauchquetschelement 31 umfasst ferner ein zentrales Quetschelement 36, das zentral zwischen den beiden Schlauchauflagen 35 angeordnet ist und auf seiner im Betriebszustand dem Schlauch 100 zugewandten Seite 31 abgerundet, vorzugsweise konvex abgerundet, ausgestaltet ist. Im Betriebszustand wird der Schlauch 100 in der Schliessstellung des Schliessorgans 2 zwischen dem Schliessstück 21 und dem zentralen Quetschelement 36 des Schlauchquetschelements 31 abgeklemmt.

Vorzugsweise werden mehrere austauschbare Schlauchquetschelemente 31 zur Verfügung gestellt, sodass für eine spezifische Anwendung das passende Schlauchquetschelement 31 entsprechend den Dimensionen des Schlauches 100, insbesondere des Aussendurchmessers und/oder des Innendurchmessers des Schlauchs 100, ausgewählt werden kann. Beispielsweise kann für einen Schlauch 100 mit einem kleineren Aussendurchmesser und/oder kleineren Innendurchmesser ein solches Schlauchquetschelement 31 verwendet werden, das ein höheres, also weiter in die Aufnahme 4 hineinreichendes zentrales Quetschelement 36 aufweist als ein Schlauchquetschelement 31, das für einen Schlauch 100 mit grösserem Aussendurchmesser und/oder Innendurchmesser vorgesehen ist.

Auch kann für verschiedene Schlauchquetschelemente 31 die Form und die Ausgestaltung der Schlauchauflagen 35 variiert werden.

Ferner ist es bevorzugt, dass ein in der axialen Richtung A bewegbares Schutzelement 25 vorgesehen ist, welches durch eine Feder 27 belastet ist, derart dass das Schutzelement 25 die beiden lateralen Öffnungen 32 überdeckt, wenn der Verschlussdeckel 3 in der in Fig. 3 dargestellten ersten Position ist. Das Schutzelement 25 umfasst zwei Seitenwände 251, die auf beiden Seiten des Schliessstücks 21 angeordnet sind, sodass sich das Schliessstück 21 zwischen den beiden Seitenwänden 251 befindet. Die Seitenwände 251 sind im Verschlussgehäuse 13 innenliegend jeweils vor einer der lateralen Öffnungen 32 angeordnet. Bezüglich der axialen Richtung A sind die Seitenwände 251 so bemessen, dass sie in der ersten Position des Verschlussdeckels 3 (Fig. 3) vorzugsweise bündig mit den lateralen Öffnungen 32 abschliessen, das heisst, die lateralen Öffnungen 32 vollständig überdecken und in axialer Richtung A nicht über die lateralen Öffnungen 32 herausragen. Falls kein Schlauch 100 in die Quetschventilvorrichtung 1 eingelegt ist, werden die beiden lateralen Öffnungen 32 also sowohl in der ersten Position als auch in der zweiten Position vollständig von den Seitenwänden 251 des Schutzelements 25 überdeckt und dienen somit als Fingerschutz, mit dem ein unbeabsichtigtes Eingreifen in die lateralen Öffnungen 32 vermieden wird.

In anderen Ausführungsformen können die Seitenwände 251 bezüglich der axialen Richtung auch etwas kürzer ausgestaltet sein, sodass sie die lateralen Öffnungen nicht vollständig verschliessen, aber vorzugsweise so weit, dass sie ein unbeabsichtigtes Eingreifen mit einem Finger verhindern.

Ferner sind Führungsschienen 26 im Verschlussgehäuse 13 vorgesehen, welche das Schutzelement 25 in axialer Richtung A führt, sodass das Schutzelement 25 in axialer Richtung A entlang der Führungsschienen 26 hin und her bewegbar ist. Zwischen dem Schutzelement 25 und dem Schliessorgan 2 ist die Feder 27 vorgesehen, welche so angeordnet ist, dass sie das Schutzelement 25 in die in Fig. 3 dargestellte Position vorspannt, in welcher die Seitenwände 251 des Schutzelements 25 die lateralen Öffnungen 32 vollständig überdecken. Zum Einlegen eines Schlauches 100 in die Aufnahme 4 muss also das Schutzelement 25 gegen die Kraft der Feder 27 derart bewegt werden, dass die Seitenwände 251 die lateralen Öffnungen 32 zumindest teilweise freigeben. Wird ein Schlauch 100 in die Aufnahme 4 eingelegt, so wird mit dem Schlauch 100 das Schutzelement 25 gegen die Kraft der Feder 27 in axialer Richtung bewegt, bis der Schlauch 100 in der Aufnahme 4 und in den lateralen Öffnung 32 liegt. Anschliessend wird der Verschlussdeckel 3 in die zweite Position gebracht und dort mittels des Sicherungselements 33 gesichert.

Das Schutzelement 25 mit den beiden Seitenwänden 251 dient ferner zur Zentrierung des Schlauches 100, insbesondere bei Schläuchen 100, die einen kleinen Aussendurchmesser aufweisen. Durch die Seitenwände 251 wird der Schlauch 100 zusätzlich fixiert, sodass auch für Schläuche 100, die einen kleinen Aussendurchmesser aufweisen, zuverlässig vermieden wird, dass sich der Schlauch 100 relativ zu der Aufnahme 4 bewegen kann, wenn der Verschlussdeckel 3 in der zweiten Position ist (siehe Fig. 4) ist. Ferner verhindert das Schutzelement 25 eine ungleichmässige Quetschung des Schlauches 100 sowie eine exzentrische Krafteinleitung in das Schliessstück 21.

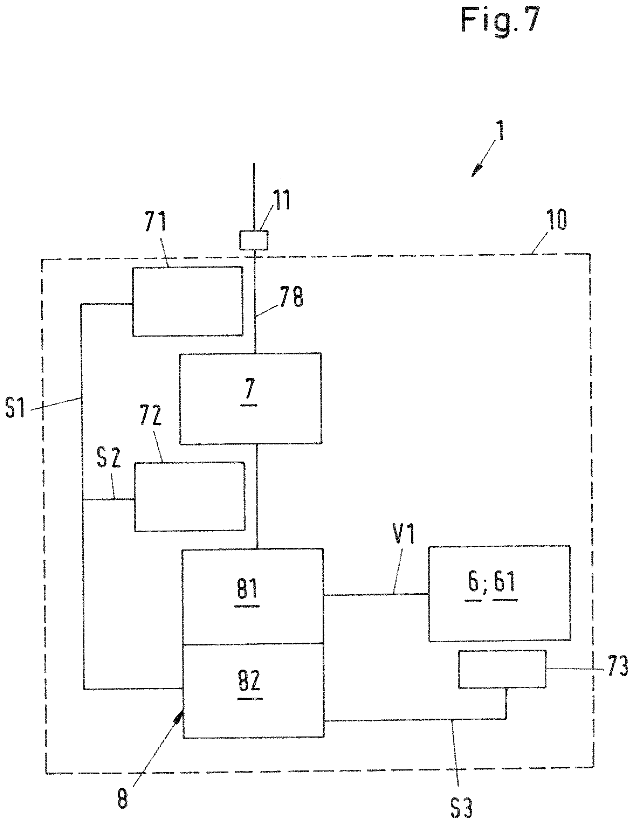

Fig. 7 zeigt in einer der Fig. 5 entsprechenden Darstellung eine erste Variante des vorangehend beschriebenen Ausführungsbeispiels. Bei dieser ersten Variante ist zusätzlich ein Messsensor 72 zur Ermittlung der im Energiespeicher 7 gespeicherten Energie vorgesehen. Der Messsensor 72, der beispielsweise als Spannungssensor ausgestaltet sein kann, ist über eine Signalleitung S2 mit der Steuereinheit 82 der Kontrolleinheit 8 signalverbunden. Der Messsensor 72 überwacht den Energiespeicher 7 und ermöglicht zumindest eine Abschätzung, ob noch ein Schaltvorgang mit der im Energiespeicher 7 vorhandenen Energie durchführbar ist. Der Messsensor 72 kann beispielsweise als Spannungsmesseinheit ausgestaltet sein, die derart angeordnet ist, dass die elektrische Spannung am Kondensator messbar ist. Aus der Spannung am Kondensator des Energiespeichers 7 kann dann die im Kondensator gespeicherte elektrische Energie zumindest abgeschätzt werden. Die im Kondensator momentan gespeicherte Energie kann dann, beispielsweise in der Steuereinheit 82, mit der für einen Schaltvorgang benötigten elektrischen Energie verglichen werden.

Als weitere Option ist es möglich, einen Positionssensor 73 zur Ermittlung der Position des Schliessorgans 2 vorzusehen. Hierzu ist jeder an sich bekannte Sensor geeignet, mit welchem die Position des Schliessorgans 2 ermittelbar ist. Insbesondere ist mit dem Positionssensor 73 erkennbar, ob sich das Schliessorgan 2 in der Offenstellung oder in der Schliessstellung befindet. Der Positionssensor 73 ist über eine Signalleitung S3 mit der Steuereinheit 82 der Kontrolleinheit 8 signalverbunden.

Wenn mithilfe des Überwachungssensors 71 eine Unterbrechung der Energieversorgung durch die externe Energiequelle detektiert wird, so überprüft die Steuereinheit 82, welches die vorgegebene Ruheposition ist. Von dem Positionssensor 73 erhält die Steuereinheit 82 die Information über die aktuelle Stellung des Schliessorgans 2, nämlich, ob sich das Schliessorgan 2 in der Offenstellung oder in der Schliessstellung befindet. Falls die aktuelle Stellung des Schliessorgans 2 mit der Ruheposition übereinstimmt, sind keine weiteren Massnahmen nötig und es wird nichts weiter unternommen. Falls die aktuelle Stellung des Schliessorgans 2 nicht der vorgegebenen Ruheposition entspricht, gibt die Steuereinheit 82 einen Befehl an die Leistungseinheit 81, dass ein Schaltvorgang auszuführen ist. Die Betätigungseinrichtung 6 wird entsprechend angesteuert, und somit wird das Schliessorgan 2 in die gewünschte Ruheposition bewegt. Mit dem Positionssensor 73 kann dann zusätzlich überprüft werden, ob das Schliessorgan 2 die vorgegebene Ruheposition erreicht hat.

Es ist natürlich nicht notwendig, aber möglich, dass sowohl ein Positionssensor 73 und ein Messsensor 72 vorgesehen sind. In anderen Ausführungsformen kann nur der Positionssensor 73 und kein Messsensor 72 vorgesehen sein. In wieder anderen Ausführungsformen kann nur der Messsensor 72 und kein Positionssensor 73 vorgesehen sein.

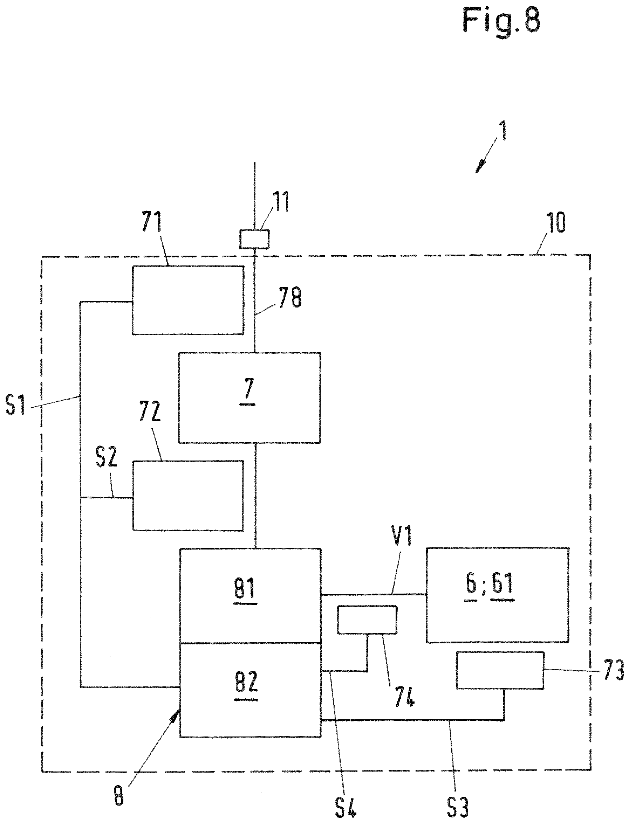

Fig. 8 zeigt in einer der Fig. 5 entsprechenden Darstellung eine zweite Variante des beschriebenen Ausführungsbeispiels. Bei dieser zweiten Variante ist im Vergleich zur ersten Variante (Fig. 7) zusätzlich ein Stromsensor 74 zur Ermittlung eines Schaltstroms vorgesehen, der über eine Signalleitung S4 mit der Steuereinheit 82 der Kontrolleinheit 8 signalverbunden ist. Der Schaltstrom ist derjenige elektrische Strom, der für den jeweiligen Schaltvorgang benötigt wird.

Der Schaltstrom kann ein Schliessstrom sein, wobei der Schliessstrom derjenige elektrische Strom ist, der für den Schaltvorgang aus der Offenstellung des Schliessorgans 2 in die Schliessstellung des Schliessorgans 2 benötigt wird.

Der Schaltstrom kann ein Öffnungsstrom sein, wobei der Öffnungsstrom derjenige elektrische Strom ist, der für den Schaltvorgang aus der Schliessstellung des Schliessorgans 2 in die Offenstellung des Schliessorgans 2 benötigt wird.

Der Stromsensor 74 ist daher so angeordnet und ausgestaltet, dass er den in die Betätigungseinrichtung 6, insbesondere den in die Spule 61 der Betätigungseinrichtung 6, eingespeisten Strom ermitteln kann. Mittels des Stromsensors 74 ist der elektrische Strom, welcher für den Schaltvorgang aus der Offenstellung in die Schliessstellung bzw. aus der Schliessstellung in die Offenstellung benötigt wird, über den gesamten Hub des Schliessorgans 2 aus der Offenstellung in die Schliessstellung bzw. aus der Schliessstellung in die Offenstellung ermittelbar.

Insbesondere ist es bei dieser Ausgestaltung möglich, dass mit der Quetschventilvorrichtung 1 eine Schlaucherkennung anhand des Schaltstroms durchgeführt wird. Hierzu können beispielsweise in der Steuereinheit 82 der Kontrolleinheit 8 in einer Zuordnungstabelle Zusammenhänge gespeichert sein, welcher Schliessstrom und/oder welcher Öffnungsstrom für welchen Schlauchtyp benötigt wird, um einen Schaltvorgang aus der Offenstellung in die Schliessstellung bzw. umgekehrt durchzuführen. Der Schlauchtyp kann dabei z.B. Informationen über das jeweilige Material des Schlauches, den Aussendurchmesser, den Innendurchmesser, die Wandstärke und gegebenenfalls noch weitere Parameter enthalten. Die Zuordnungstabelle ist dann beispielsweise eine mehrdimensionale Tabelle, in welcher die Zusammenhänge zwischen denjenigen Grössen, welche den Schlauchtyp bestimmen, und den benötigten Schaltströmen hinterlegt sind. Anhand des messtechnisch erfassten Schaltstroms und gegebenenfalls dem Signal des Positionssensors 73 ist es dann möglich, den in die Quetschventilvorrichtung 1 eigelegten Schlauch 100 zu identifizieren.

Ferner ist es möglich, anhand eines durchgeführten Schaltvorgangs aus der Schliessstellung in die Offenstellung oder aus der Offenstellung in die Schliessstellung und der messtechnischen Erfassung des dafür benötigten Schaltstroms zu erkennen, ob ein Schlauch 100 in die Aufnahme 4 der Quetschventilvorrichtung 1 eingelegt ist oder nicht.

Selbstverständlich sind auch solche Ausgestaltungen möglich, bei denen die Schlaucherkennung nur anhand des Schliessstroms oder nur anhand des Öffnungsstroms durchgeführt wird. Auch kann für die Schlaucherkennung eine Mehrzahl von Schaltvorgängen durchgeführt werden und dabei jeweils der benötige Öffnungsstrom bzw. Schliessstrom gemessen werden. Beispielsweise kann ein Schaltvorgang aus der Offenstellung in die Schliessstellung und anschliessend ein Schaltvorgang aus der Schliessstellung in die Offenstellung durchgeführt werden.

Die Schlaucherkennung kann insbesondere auch an einem Schlauch 100 durchgeführt werden, der noch nicht von einem Fluid durchströmt wird, also beispielsweise an einem leeren Schlauch 100.

## Patentansprüche

1. Quetschventilvorrichtung zum Abklemmen eines Schlauches in einem Fluidsystem, mit einem in einer axialen Richtung (A) bewegbar angeordneten Schliessorgan (2), welches ein Schliessstück (21) zum Abklemmen des Schlauches (100) umfasst, mit einer permanentmagnetischen Halteeinrichtung (5), welche derart ausgestaltet ist, dass sie das Schliessorgan (2) mittels einer permanentmagnetischen Kraft in zwei verschiedenen stabilen Gleichgewichtslagen halten kann, nämlich in einer Offenstellung und in einer Schliessstellung, ohne dass der permanentmagnetischen Halteeinrichtung (5) für das Halten in der jeweiligen Gleichgewichtslage Energie zugeführt werden muss, sowie mit einer elektromagnetischen Betätigungseinrichtung (6) zum Durchführen eines Schaltvorgangs, mit welchem das Schliessorgan (2) aus der Offenstellung in die Schliessstellung oder aus der Schliessstellung in die Offenstellung bewegt wird, **dadurch gekennzeichnet, dass** ein Energiespeicher (7) und eine Kontrolleinheit (8) vorgesehen sind, wobei in dem Energiespeicher (7) eine elektrische Energie speicherbar ist, die mindestens zum Durchführen eines Schaltvorgangs ausreicht, wobei der Kontrolleinheit (8) eine Ruheposition vorgebbar ist, welche die Offenstellung oder die Schliessstellung ist, und wobei die Kontrolleinheit (8) einen Schaltvorgang auslösen kann, mit welchem das Schliessorgan (2) mittels der im Energiespeicher (7) gespeicherten Energie in die Ruheposition gebracht werden kann.

2. Quetschventilvorrichtung nach Anspruch 1, wobei das Schliessorgan (2), die Halteeinrichtung (5), die Betätigungseinrichtung (6), der Energiespeicher (7) und die Kontrolleinrichtung (8) in einem gemeinsamen Gehäuse (10) angeordnet sind, wobei das Gehäuse (10) einen Anschluss (11) zum Verbinden mit einer externen Energiequelle aufweist.

3. Quetschventilvorrichtung nach Anspruch 2, wobei in dem gemeinsamen Gehäuse (10) ein Überwachungssensor (71) vorgesehen ist, mit welchem eine Unterbrechung der Energieversorgung durch die externe Energiequelle detektierbar ist.

4. Quetschventilvorrichtung nach einem der vorangehenden Ansprüche, wobei die permanentmagnetische Halteeinrichtung (5) einen permanentmagnetischen Ring (51) umfasst, welcher das Schliessorgan (2) umgibt.

5. Quetschventilvorrichtung nach einem der vorangehenden Ansprüche, wobei der Energiespeicher (7) als Zwischenkreis für die Energieversorgung der Betätigungseinrichtung (6) ausgestaltet ist.

6. Quetschventilvorrichtung nach einem der vorangehenden Ansprüche, wobei ein Positionssensor (73) zur Ermittlung der Position des Schliessorgans (2) vorgesehen ist.

7. Quetschventilvorrichtung nach einem der vorangehenden Ansprüche, wobei ein Messsensor (72) zur Ermittlung der im Energiespeicher (7) gespeicherten elektrischen Energie vorgesehen ist.

8. Quetschventilvorrichtung nach einem der vorangehenden Ansprüche, wobei ein Stromsensor (74) zur Ermittlung eines Schaltstroms vorgesehen ist, wobei der Schaltstrom derjenige Strom ist, der für den Schaltvorgang benötigt wird.

9. Quetschventilvorrichtung nach Anspruch 8, wobei eine Schlaucherkennung anhand des Schaltstroms durchführbar ist.

10. Quetschventilvorrichtung nach einem der Ansprüche 2-9, wobei im Gehäuse zwei laterale Öffnungen (32) zum Aufnehmen eines Schlauches (100) vorgesehen sind, wobei ein Verschlussdeckel (3) vorgesehen ist, der gelenkig mit dem Gehäuse (10) verbunden ist, und zwischen einer ersten Position und einer zweiten Position hin und her bewegbar ist, wobei in der ersten Position der Schlauch (100) in die lateralen Öffnungen (32) einlegbar ist, und wobei in der zweiten Position der Schlauch (100) relativ zum Gehäuse (10) der Quetschventilvorrichtung (1) fixiert ist.

11. Quetschventilvorrichtung nach Anspruch 10, wobei im Verschlussdeckel (3) ein Schlauchquetschelement (31) zum Zusammenwirken mit dem Schliessstück (21) des Schliessorgans (2) angeordnet ist, derart, dass der Schlauch (100) zwischen dem Schliessstück (21) und dem Schlauchquetschelement (31) einklemmbar ist, wenn der Verschlussdeckel (3) in der zweiten Position ist.

12. Quetschventilvorrichtung nach Anspruch 11, wobei das Schlauchquetschelement (31) austauschbar ausgestaltet und angeordnet ist.

13. Quetschvorrichtung nach Anspruch 12, wobei im Verschlussdeckel (3) eine Ausnehmung (34) vorgesehen ist, in welche das Schlauchquetschelement (31) einschiebbar ist.

14. Quetschventilvorrichtung nach einem der Ansprüche 8-11, wobei ein in der axialen Richtung (A) bewegbares Schutzelement (25) vorgesehen ist, welches durch eine Feder (27) belastet ist, derart, dass das Schutzelement (25) die beiden lateralen Öffnungen (32) überdeckt, wenn der Verschlussdeckel (3) in der ersten Position oder in der zweiten Position ist, und kein Schlauch (100) in die Quetschventilvorrichtung eingelegt ist.

15. Quetschventilvorrichtung nach einem der Ansprüche 10-14, wobei am Gehäuse ein Sicherungselement (33) vorgesehen ist, mit welchem der Verschlussdeckel (3) in der zweiten Position fixiert werden kann.
